# EUROPÄISCHE PATENTSCHRIFT

(11) **EP 1 114 044 B1**
(45) Veröffentlichungstag und Bekanntmachung des Hinweises auf die Patenterteilung: **28.08.2002**
(21) Anmeldenummer: 99947361.4
(22) Anmeldetag: 16.09.1999
(51) Int. Cl.: C07D 323/06

(54) **ABTRENNUNG VON TRIOXAN AUS FLÜSSIGEN GEMISCHEN**
SEPARATION OF TRIOXAN FROM LIQUID MIXTURES
SEPARATION DE TRIOXANE CONTENU DANS DES MELANGES LIQUIDES

(30) Priorität: 17.09.1998 DE 19842579
(43) Veröffentlichungstag der Anmeldung: 11.07.2001
(73) Patentinhaber: Ticona GmbH, 65451 Kelsterbach (DE)
(72) Erfinder: SCHWEERS, Elke, D-69812 Bad Soden (DE); REICHL, Albert, D-65929 Frankfurt (DE); ROSENBERG, Michael, D-65527 Niedernhausen (DE); HEFFELS, Stefan, D-65817 Eppstein (DE)
(86) Internationale Anmeldenummer: EP9906843
(87) Internationale Veröffentlichungsnummer: WO00017188

(56) Entgegenhaltungen:
- US-A- 5 061 349

## Beschreibung

Die Erfindung betrifft die Abtrennung von Trioxan aus einem flüssigen Gemisch enthaltend Trioxan, Formaldehyd, Alkohol, aus Formaldehyd und Alkohol gebildete Hemiformale sowie geringen Mengen an Nebenkomponenten. Zusätzlich oder alternativ zum Alkohol und den Hemiformalen können in dem Gemisch auch Wasser sowie aus Formaldehyd und Wasser gebildete Reaktionsprodukte enthalten sein.

Zur Herstellung des technischen Kunststoffs Polyacetal, insbesondere von Polyoxymethylen (POM), wird hoch reines Trioxan benötigt. Die Qualität des Kunststoffes, d.h. der erreichbare Polymerisationsgrad, wird neben den Polymerisationsbedingungen vorrangig durch die Reinheit des Trioxans bestimmt.

Es sind verschiedene Verfahren zur Herstellung von Trioxan bekannt (z. B. homogen oder heterogen katalysiert aus wäßrigen Formaldehydlösungen (AT 252913) oder heterogen katalysiert aus gasförmigem Formaldehyd an Heteropolysäuren (EP 0606056). Unabhängig vom Herstellverfahren fällt Trioxan im allgemeinen nicht als reiner Stoff, sondern immer im Gemisch mit nicht umgesetztem Formaldehyd und anderen Stoffen wie Alkohol, Wasser und geringen Mengen anderer Komponenten, sogenannter Nebenkomponenten wie Methanol, Methylformiat, Methylal, Ameisensäure, Dioxolan und Tetroxan an. Zur Verwendung des Trioxans in der Polymerisation muß dieses insbesondere vom Formaldehyd abgetrennt werden und darf nur geringe Mengen an Nebenkomponenten enthalten.

Es ist eine Vielzahl von Literatur bekannt, die die Abtrennung von Trioxan aus wäßrigen Formaldehyd-haltigen Gemischen betrifft. Die Trennung gasförmiger Gemische aus Formaldehyd und Trioxan wird nur an wenigen Stellen beschrieben.

Die Trennung aus wäßriger Lösung erfolgt bisher insbesondere über die Destillation (AT 252913 und JP 83-171278). Bei der Destillation wird nicht umgesetzter Formaldehyd häufig wieder in den Reaktor zurückgeführt und dort weiter zu Trioxan umgesetzt. Ein Grenzwert für die Anreicherung des Trioxans durch die destillative Abtrennung ergibt sich durch ein bei 92°C und 1 bar siedendes Azeotrop von Trioxan mit Wasser, welches im allgemeinen einen Trioxan-Anteil von ca 70 Gew.-% aufweist. Ein Nachteil dieses Verfahrens ist ferner in einer möglichen Feststoffbildung durch Polymerisation von Formaldehyd oder Bildung von para-Formaldehyd, vor allem im Bereich des Kolonnenkopfes, zu sehen. Zur Vermeidung derselben müssen alle Apparateteile entweder auf Temperaturen über 100°C (z. B. bei 1 bar Formaldehyd-Partialdruck) aufgeheizt oder mit einer Flüssigkeit benetzt werden.

Ein weiteres Verfahren zur Trennung von Formaldehyd und Trioxan aus wäßrigen Lösungen besteht in der Extraktion des Trioxans mit organischen Lösungsmitteln, in denen Trioxan eine höhere physikalische Löslichkeit besitzt als der Formaldehyd. Diese Verfahren wurden ausschließlich zur Abtrennung des Trioxans aus der wäßrigen Phase eingesetzt. Als organische Extraktionsmittel werden beispielsweise gesättigte aliphatische oder aromatische Kohlenwasserstoffe oder Halogenkohlenwasserstoffe verwendet (EP 0583907), die wenig oder gar nicht mit Wasser mischbar sind. Ein Nachteil der Extraktion ist, daß mit dem organischen Lösungsmittel ein zusätzlicher Stoff in das Verfahren eingebracht wird, wodurch eine anschließende Aufarbeitung auch der organischen Phase erforderlich wird. Ein weiterer Nachteil besteht darin, daß mitunter auch große Teile des Trioxans in der wäßrigen Phase verbleiben. Große Mengen an Trioxan müssen daher im Kreis geführt werden oder gehen beim Aufarbeitungsverfahren verloren.

Als weitere Möglichkeit der selektiven Trennung aus einer wäßrigen Formaldehyd-Trioxan-Phase wird die Kristallisation des Trioxans beschrieben (DE 3508668). Die Trioxankonzentration in dem wäßrigen Gemisch muß dabei größer als 50 Gew.-% sein.

Bei der Herstellung von Trioxan durch Trimerisierung von Formaldehyd aus wäßrigen Formalinlösungen werden in der Regel die oben genannten Verfahren der azeotropen Destillation, Extraktion und gegebenenfalls der Kristallisation in geeigneter Weise miteinander verknüpft, um Trioxan in der geforderten hohen Reinheit zu erhalten.

Für die Trennung von gasförmigen Gemischen von Formaldehyd und Trioxan wurde mehrfach die selektive Absorption einer Spezies eingesetzt. Dabei wird im allgemeinen entweder der Formaldehyd chemisorbiert und das Trioxan in der Gasphase belassen (GB 1245990) oder es erfolgt umgekehrt eine selektive Physisorption des Trioxans (EP 0680959). Da keine flüssige Phase gefunden wurde, in der entweder nur der Formaldehyd oder nur das Trioxan löslich ist, werden dabei auch Anteile der jeweils anderen Spezies gebunden. Deshalb können mit diesem Verfahren die für die Polymerisation nötigen Reinheiten nicht erzieit werden. Weiterhin entstehen große Verluste am Wertstoff Trioxan.

Die Trennung eines gasförmig und wasserarm vorliegenden Gemisches aus Formaldehyd und Trioxan durch Absorption des Trioxans in einem Alkohol und anschließender Kristallisation aus der alkoholischen Lösung wird in der noch nicht veröffentlichten deutschen Patentanmeldung Nr. 19833620.9 beschrieben. Dieses Verfahren bildet einen möglichen Schritt in einem neuen Prozeß zur Herstellung von Trioxan aus Methanol, bestehend aus den Schritten nicht oxidative Dehydrierung (DE 3920811), Formaldehydabtrennung (noch nicht veröffentlichte deutsche Patentanmeldungen Nr. 19747647.3 und 19748380.1), Formaldehydtrimerisierung (EP 0606056 und EP 0691388) und Trioxanabtrennung (z. B. nach der deutschen Patentanmeldung Nr. 19833620.9).

Es bestand nun die Aufgabe, ein alternatives Verfahren zur Abtrennung von Trioxan aus einem flüssigen Gemisch enthaltend Trioxan, Formaldehyd, Alkohol, aus Formaldehyd und dem Alkohol gebildete Hemiformale, Nebenkomponenten sowie, zusätzlich oder alternativ zum Alkohol und den Hemiformalen, Wasser und aus Formaldehyd und Wasser gebildete Reaktionsprodukte zu finden, wobei das Trioxan in hoher Reinheit gewonnen werden sollte. Die anderen im Gemisch enthaltenen Wertstoffe Formaldehyd und gegebenenfalls der Alkohol sollten dabei möglichst in anderen verwertbaren Produktströmen enthalten sein.

Diese Aufgabe wird erfindungsgemäß gelöst, indem Trioxan aus dem flüssigen Gemisch in geeigneter Weise möglichst selektiv in die Dampfphase überführt und anschließend selektiv durch Abkühlung und daraus folgender Kondensation oder Desublimation flüssig bzw. fest abgetrennt wird.
Die Erfindung betrifft daher ein Verfahren zur Gewinnung von Trioxan, wobei aus einem flüssigen Gemisch enthaltend Trioxan, Formaldehyd, Alkohol, aus dem Formaldehyd und dem Alkohol gebildete Hemiformale, übliche bei der Herstellung von Trioxan anfallende geringe Mengen an leichter- und schwerersiedenden Nebenkomponenten sowie, zusätzlich oder alternativ zum Alkohol und den Hemiformalen, Wasser das Trioxan durch Verdunstung oder Verdampfung in die Gasphase überführt und anschließend durch Kondensation in einen flüssigen Zustand gebracht und als Kondensat gewonnen oder durch Desublimation in einen festen Zustand gebracht und als Desublimat gewonnen wird.

Die Erfindung betrifft auch die Verwendung des nach dem erfindungsgemäßen Verfahren gewonnenen Trioxan-Kondensats oder -Desublimats, das durch seine Zusammensetzung und einen Gehalt an Trioxan von mindestens 80 Gew.-% charakterisiert ist, zur Herstellung von Polymeren und Brennstoffen oder zur Gewinnung von Formaldehyd durch Depolymerisation.

Übliche Nebenkomponenten sind beispielsweise Methanol, Methylformiat, Tetroxan, Dioxolan, Trioxyether und Spuren von Ameisensäure. Bei dem im flüssigen Ausgangsgemisch enthaltenen Alkohol, der mit dem Formaldehyd teilweise Hemiformale bilden kann, handelt es sich vorzugsweise um einen einwertigen Alkohol, z. B. Cyclohexanol, Methanol, Propanol oder Butanol. Es ist aber auch der Einsatz anderer, auch mehrwertiger Alkohole wie Glyzerol, di-Ethylen-Glykol, tri-Ethylen-Glykol, tri-Ethanolamin, Butantriol und Pentantriol möglich. Wahlweise kann auch ein Gemisch von Alkoholen verwendet werden. Vorzugsweise sollte der Alkohol einen höheren Siedepunkt und einen niedrigeren Schmelzpunkt als Trioxan aufweisen.

Das erfindungsgemäße Verfahren löst im Gegensatz zur deutschen Patentanmeldung Nr. 19833620.9 und zu DE 3508668 insbesondere die Aufgabe der Trennung eines flüssig und wasserarm vorliegenden Gemisches, wie es z. B. in der Absorptionsstufe nach dem Verfahren gemäß der deutschen Patentanmeldung Nr. 19833620.9 und damit in dem oben genannten neuen Prozeß zur Herstellung von Trioxan aus Methanol anfällt. Das Verfahren ist aber auch auf Gemische mit einem höheren Gehalt an Wasser anwendbar.
Überraschend wurde dabei gefunden, daß mit dem erfindungsgemäßen Verfahren eine hohe Aufkonzentration von Trioxan auch bei einer sehr geringen Ausgangskonzentration des Trioxans im flüssigen Gemisch erzielt werden kann.

Zur Gewinnung des Trioxans nach dem erfindungsgemäßen Verfahren wird das Trioxan zunächst bei einer Temperatur im Bereich von 20 bis 200°C, bevorzugt im Bereich von 50 bis 100°C in einem geeigneten Apparat mit oder ohne Trägergas aus dem flüssigen Gemisch in die Dampfphase überführt. Anschließend wird das Trioxan bei einer Temperatur im Bereich von -20 und 113°C, bevorzugt im Bereich von 20 bis 40°C aus der Dampfphase in einen flüssigen oder festen Aggregatzustand überführt, d.h. kondensiert oder desublimiert. Das Verfahren läßt sich bei Unterdruck, Normaldruck oder Überdruck durchführen, wobei vorzugsweise Normaldruck angelegt wird.

Im Unterschied zu DE 3508668 erfolgt die Abtrennung beim erfindungsgemäßen Verfahren vorzugsweise aus einem wasserarmen flüssigen Gemisch. Wasserarm bedeutet dabei, daß das Gemisch entweder gar kein Wasser, d.h. 0 Gew.-%, oder aber maximal 5 Gew.-%, bevorzugt maximal 3 Gew.-% Wasser enthält. Hierin liegt ein besonderer Vorteil gegenüber dem Stand der Technik da durch den geringen Gehalt an Wasser im Prozeß deutlich weniger Heizenergie erforderlich ist und eine Aufarbeitung von wäßrigen Gemischen nahezu entfällt. Auch die im Verfahren gemäß DE 3508668 vorliegende Beschränkung auf Gemische mit einem Trioxan-Anteil größer 50 % ist beim erfindungsgemäßen Verfahren nicht gegeben. Das erfindungsgemäße Verfahren läßt sich darüber hinaus aber auch für die Aufreinigung von Trioxan aus wäßrigen Gemischen einsetzen, die einen deutlich höheren Wasseranteil als 5 % aufweisen.

Im Unterschied zu dem in der deutschen Patentanmeldung Nr. 19833620.9 beschriebenen Verfahren einer Abtrennung von Trioxan durch Kristallisation in einer flüssigen Lösung, entweder an gekühlten Wänden oder in Suspension, wird beim erfindungsgemäßen Verfahren das Trioxan erst selektiv verdunstet oder verdampft und anschließend durch Kondensation oder Desublimation in einen flüssigen Zustand (Kondensat) oder - bevorzugt - in einen festen Zustand (Desublimat) überführt. Es lassen sich dadurch höhere Reinheiten und Ausbeuten als bei dem Verfahren nach der deutschen Patentanmeldung Nr. 19833620.9 erzielen.

Des weiteren ist das erfindungsgemäße Verfahren energetisch und damit hinsichtlich der Betriebskosten vorteilhafter, da bei der Kristallisation nach der deutschen Patentanmeldung Nr. 19833620.9 Kälte zum Einsatz kommt, was beim erfindungsgemäßen Verfahren nicht notwendig ist. Auch die zu erwartenden Apparatekosten liegen beim erfindungsgemäßen Verfahren niedriger als bei der in der deutschen Patentanmeldung Nr. 19833620.9 beschriebenen Schichtkristallisation.

Für die Überführung des Trioxans aus dem flüssigen Gemisch in die Dampfphase ist es beim erfindungsgemäßen Verfahren vorteilhaft, jedoch nicht zwingend, wenn Trioxan (Siedepunkt bei Normaldruck 113°C) gegenüber den anderen Komponenten einen vergleichsweise hohen Dampfdruck besitzt. Wird die Abkühlung als Desublimation ausgeführt, so erweist sich der vergleichsweise hohe Schmelzpunkt (ca. 62°C) von Trioxan als besonders vorteilhaft. Durch die hiermit mögliche selektive Abtrennung von Trioxan aus der Dampfphase kann die Dampfphase auch leichter- und schwerersiedende Komponenten enthalten.

Es sind eine Reihe von Möglichkeiten gegeben, beim erfindungsgemäßen Verfahren das Trioxan aus dem flüssigen Gemisch in die Dampfphase zu überführen. So läßt sich beispielsweise bei genügend hohem Trioxan-Anteil in der Mischung eine einfache Verdampfung bei Normaldruck durch Temperaturerhöhung durchführen. Nachteilig hierbei ist die vergleichsweise hohe thermische Belastung, die eine Abspaltung von Formaldehyd aus dem Hemiformal nach sich ziehen kann.

Die Überführung von Trioxan in die Dampfphase kann auch durch Verdunstung oder Verdampfung unter Vakuum geschehen. Vorteilhaft sind hierbei die niedrigen Temperaturen, nachteilig sind mögliche Leckageprobleme sowie der erforderliche Aufwand zur Bereitstellung des Vakuums.

Als besonders vorteilhaft erweist sich im erfindungsgemäßen Verfahren der Einsatz eines Trägergases, das auch mit Trioxan vorbeladen sein kann. Mit diesem Trägergas wird Trioxan aus dem flüssigen Gemisch ausgestrippt. Es lassen sich hierbei sowohl die vorteilhaften geringeren Temperaturen als auch der Betrieb unter Normaldruck realisieren. Zur Vermeidung eines zu großen Bedarfes an Gas ist jedoch ein Verdichter einzusetzen, mit welchem das Trägergas zwischen der Verdunstungs- bzw. Verdampfungsstufe und der Kondensations- bzw. Desublimationsstufe im Kreis geführt wird. Als Trägergase eignen sich beispielsweise inerte Gase wie Stickstoff und Argon.

Bei thermisch schonender Betriebsweise der Verdunstungs- bzw. Verdampfungsstufe im erfindungsgemäßen Verfahren unter moderaten Temperaturen erweist es sich als besonders vorteilhaft, daß nahezu keine Abspaltung von Formaldehyd aus dem Hemiformal auftritt. Hierdurch werden Probleme mit Belagbildung, z. B. durch Ausfall von para-Formaldehyd aus der Gasphase, vermieden.

Zur Überführung von Trioxan in die Dampfphase ist beim erfindungsgemäßen Verfahren eine Reihe von Apparaten einsetzbar. So ist dieser Verfahrensschritt beispielsweise in Verdampfern, wie Fallfilm- oder Dünnschichtverdampfern, durchführbar. Es sind aber auch von außen beheizte Kolonnen denkbar, die als Sprühtürme ausgeführt sein können und wahlweise Einbauten wie Böden oder strukturierte Packungen aufweisen. Weiterhin läßt sich ein beheiztes Rührgefäß verwenden oder aber auch eine Blasensäule, insbesondere wenn ein Trägergas zum Einsatz kommt. Denkbar ist auch der Einsatz von Flash-Apparaten, in denen überhitzte Lösungen auf einen niedrigeren Druck entspannt werden.

Bevorzugt sind solche Apparaturen zu verwenden, die als Standardapparate bei einfacher Bauart und kontinuierlicher Betriebsweise eine genügend große Phasengrenzfläche Dampf/Flüssigkeit und hohe Durchsätze aufweisen. Diese Anforderungen werden z. B. von Verdampfer wie Fallfilm- oder Dünnschichtverdampfer erfüllt.

Die Verdunstung oder Verdampfung kann beim erfindungsgemäßen Verfahren bei Temperaturen im Bereich von 20 bis 200°C erfolgen, im Unterdruckbereich gegebenenfalls auch bei tieferen, im Überdruckbereich gegebenenfalls auch bei höheren Temperaturen, bevorzugt jedoch bei einer Temperatur von 50 bis 100°C. Der aus der Verdunstungs- bzw. Verdampfungsstufe ablaufende flüssige Strom, der an Trioxan verarmt ist, aber die Wertstoffe Formaldehyd, Alkohol und Hemiformale enthält, läßt sich in eine frühere Stufe des oben beschriebenen Gesamtprozesses zurückführen oder anderweitig, z. B. zur Gewinnung der darin enthaltenen Wertstoffe, verwenden. Des weiteren ist denkbar, den Strom teilweise oder vollständig in die Verdunstungs- bzw. Verdampfungsstufe zurückzuführen. Im letzteren Fall liegt dabei vorzugsweise eine diskontinuierliche Betriebsweise vor.

Durch die Kondensation oder Desublimation des Trioxans aus der Dampfphase kann beim erfindungsgemäßen Verfahren das Trioxan wahlweise im flüssigen oder festen Zustand gewonnen werden. Soll das Kondensat flüssig bleiben, so müssen unter Beachtung des Schmelzpunktes von Trioxan in der Kondensationsstufe relativ hohe Temperaturen eingestellt werden, wodurch jedoch der Wärme- und damit der Stoffübergang und die Abscheidungsrate vermindert wird. Als Kondensatoren lassen sich hierbei alle bekannten Arten von Wärmetauschern mit Phasenübergang dampfförmig-flüssig einsetzen, nur beispielsweise seien hier Rohrbündelwärmetauscher genannt.

Das Trioxan läßt sich aus der mit ihm beladenen Gasphase auch durch Kontakt mit einer kalten Flüssigkeit kondensieren oder absorbieren. Hierfür eignen sich ebenfalls z. B. Fallfilmkondensatoren, Blasensäulen oder Rührgefäße, die mit entsprechend kalten Flüssigkeiten bzw. Lösungsmitteln betrieben werden. Nachteilig bei diesem Aufbau ist allerdings die anschließend notwendige Aufreinigung, d.h. die Abtrennung des Trioxans aus der Flüssigkeit.

Die Kondensation bis zur flüssigen Phase kann beim erfindungsgemäßen Verfahren bei Temperaturen im Bereich von 30 bis 113°C stattfinden, bei Kondensation unter Überdruck gegebenenfalls auch bei höheren Temperaturen, bevorzugt jedoch bei Temperaturen im Bereich von 30 bis 75°C.

Es ist jedoch vorteilhaft, beim erfindungsgemäßen Verfahren unter Ausnutzung des hohen Schmelzpunktes von Trioxan die Desublimation anzuwenden. Diese kann hierbei sowohl an starren, gekühlten Wänden als auch in einer bewegten Zone, z. B. einem Fließbett oder einer Wirbelschicht, erfolgen. Ein weiterer Vorteil der Desublimation liegt darin, daß durch Variation der Prozeßparameter eine gezielte Formgebung des Trioxan-Desublimats, z. B. die Abscheidung in einer gewünschten Teilchengröße, vorgenommen werden kann. Der Vorgang der Desublimation läßt sich beim erfindungsgemäßen Verfahren durch geeignete Maßnahmen, wie Zumischen eines kalten Gases oder Einspritzen eines niedrig siedenden Lösungsmittels, unterstützen.

Werden zur Desublimation starre Wände eingesetzt, so kann das Desublimat von ihnen entweder durch Aufschmelzen oder durch mechanisches Reinigen wie Abkratzen oder Abschaben entfernt werden. Dabei ist die mechanische Reinigung vorteilhafter, sofern das Produkt danach weiter flüssig zu verarbeiten ist, da keine zusätzliche Energie, wie z. B. zum Aufschmelzen benötigt, zugeführt werden muß und weil das Verfahren kontinuierlich betrieben werden kann. Als Apparat kann z. B. ein Kratzkühler eingesetzt werden. Wegen der relativ kleinen Wärmeübergangskoeffizienten beim Phasenübergang dampfförmig-fest sind im allgemeinen große Austauschflächen erforderlich. Unter diesem Aspekt ist der Einsatz von Platten- oder Rippenrohrwärmetauschern besonders vorteilhaft, von denen der feste Belag durch Aufschmelzen oder durch Sublimation, vorzugsweise unterstützt durch Temperaturerhöhung und Druckabsenkung, entfernt werden kann. Anstelle von Platten- oder Rippenrohrwärmetauschern sind auch andere Arten von Wärmetauschern, z. B. Rohrbündelwärmetauscher, einsetzbar. Der Betrieb erfolgt im allgemeinen taktweise, d.h. in einem kontinuierlichen Prozeß kommen mindestens zwei Wärmetauscher zum Einsatz.

Wird die Desublimation in einem Fließbett oder einer Wirbelschicht durchgeführt, so lassen sich höhere Wärmeübergangskoeffizienten bei kontinuierlicher Betriebsweise erzielen. Die Fließbett-Desublimation ist mit oder ohne Zugabe eines Fließmittels durchführbar, bevorzugt auch unter Zugabe eines gekühlten Gases. Als Apparate sind z. B. Wirbelschichttrockner einsetzbar; gegebenenfalls ist eine Filtration oder Siebung der Partikel nachgeschaltet.

Die Desublimation kann beim erfindungsgemäßen Verfahren bei Temperaturen im Bereich von -20 bis 70°C erfolgen, unter Umständen auch bei tieferen Temperaturen, bevorzugt jedoch bei Temperaturen im Bereich von 20 bis 40°C, da hierfür keine Kälteerzeugung erforderlich ist, was einen großen Vorteil des erfindungsgemäßen Verfahrens darstellt. Ist ein Aufschmelzen erforderlich, so läßt sich dies bei Temperaturen im Bereich von 30 bis 113°C durchführen, im Überdruckbereich auch bei höheren Temperaturen, bevorzugt jedoch bei Temperaturen im Bereich von 60 bis 80°C.
Eine Möglichkeit zur Durchführung der Verfahrensschritte Verdunstung bzw. Verdampfung und Kondensation bzw. Desublimation in einem einzigen Apparat ergibt sich bei Einsatz eines Kurzwegverdampfers, bei dem die Verdampfungs- und Kondensationsflächen konzentrisch angeordnet sind. Mit dem Kurzwegverdampfer ist ein besonders schonender Umgang mit dem Produkt möglich. Wird die Kondensationsfläche als Desublimator eingesetzt, so ist ein taktweiser Betrieb erforderlich. Der Kurzwegverdampfer kann auch als Molekular-Kondensator bzw. - Desublimator ausgeführt sein.

Weist das aus dem flüssigen Gemisch nach einer Verdunstungs- bzw. Verdampfungsstufe und Kondensations- bzw. Desublimationsstufe abgetrennte Trioxan noch nicht die gewünschte Reinheit auf, so läßt sich bei dem erfindungsgemäßen Verfahren eine mehrstufige Betriebsweise mit entsprechenden Rückführungen realisieren. Es ist weiterhin denkbar, daß das nach einer Stufe vorliegende Produkt mit Hilfe anderer Trennverfahren feingereinigt wird.

Das nach dem erfindungsgemäßen Verfahren gewonnene Trioxan enthält im allgemeinen 2 bis 4 Gew.-% Formaldehyd, 4 bis 8 Gew.-% Alkohol und mehr als 80 Gew.-%, vorzugsweise 85 bis 90 Gew.-% Trioxan. Es eignet sich für alle bekannten Einsatzgebiete, wie die Polymerisation zu Kunststoffen wie Polyacetale, insbesondere Polyoxymethylen, die Herstellung von Brennstoffen und die Depolymerisation zu Formaldehyd, wodurch eine Weiterverwendung für alle bekannten Formaldehyd-Reaktionen ermöglicht wird, insbesondere wenn dafür sehr reiner Formaldehyd nötig ist.

Insgesamt läßt sich mit dem erfindungsgemäßen Verfahren ein Trioxan-Kondensat oder -Desublimat gewinnen, dessen Gehalt an Trioxan dem 2,5-fachen oder mehr des Trioxan-Anteils im flüssigen Ausgangsgemisch entspricht. Ist der Trioxan-Anteil im flüssigen Gemisch allerdings äußerst niedrig, z. B. nur 5 Gew.-%, so erhält man im Kondensat oder Desublimat zwar keinen Trioxan-Anteil von 80 Gew.-% und mehr. Es läßt sich aber dennoch eine Anreicherung des Trioxans um das 10-fache, d.h. auf ca. 50 bis 65 Gew.-% erreichen.

Das erfindungsgemäße Verfahren zeichnet sich gegenüber den bisher eingesetzten Verfahren durch folgende Vorteile aus:
- hohe Reinheiten und Ausbeuten an Trioxan,
- bei wäßrigen Gemischen im Gegensatz zur Destillation keine Beschränkung durch das Azeotrop Wasser/Trioxan,
- einfache apparative Umsetzung mit Standardaggregaten möglich,
- geringe Anteile an Formaldehyd schon nach einer Prozeßstufe und damit günstige Voraussetzungen für eine Weiterverarbeitung,
- sehr geringe Formaldehyd-Konzentrationen in der Gasphase und damit Vermeidung der Feststoff- bzw. Belagbildung (z. B. para-Formaldehyd),
- durch moderate Temperaturen thermisch schonend und energetisch vorteilhaft in der Verdunstungs- bzw. Verdampfungsstufe; dadurch Vermeidung der Abspaltung von gasförmigem Formaldehyd,
- energetisch vorteilhaft in der Kondensations- bzw. Desublimationsstufe durch die Verwendbarkeit von Flußwasser, Rückkühlwasser oder Umgebungsluft zur Kühlung unter Vermeidung von vergleichsweise teurer Kälteerzeugung,
- zusätzlich energetisch vorteilhaft durch interne Energierückgewinnung,
- hohe Betriebssicherheit,
- keine stofflichen Hilfsmittel nötig, die aufwendig abgetrennt werden müssen,
- keine Verlustströme: Ablauf aus der Verdunster- bzw. Verdampferstufe an anderer Stelle im Verfahren einsetzbar,
- kein Anfall einer Vorlauffraktion aus einem Abschwitzvorgang in der Kondensations- bzw. Desublimationsstufe.

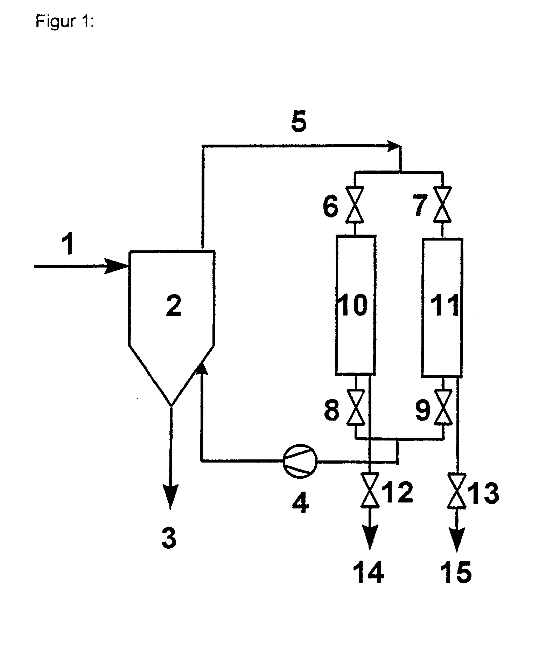

Eine mögliche apparative Umsetzung des erfindungsgemäßen Verfahrens wird im folgenden anhand von Figur 1 erläutert.

Das flüssige Ausgangsgemisch 1, das neben Trioxan auch Formaldehyd, einen Alkohol (bevorzugt mit einem höheren Siedepunkt als Trioxan, wie z. B. Cyclohexanol), Hemiformale und Nebenkomponenten, sowie zusätzlich bzw. alternativ zu Alkohol und Hemiformal noch Wasser enthält, wird dem Apparat zur Überführung des Trioxans in die dampfförmige Phase zugeleitet, der hier als Verdampfer 2 ausgeführt ist. Die Flüssigkeit durchströmt den Verdampfer 2 von oben nach unten und tritt unten als Strom 3, der an Trioxan verarmt ist, wieder aus. Der Verdichter 4 fördert einen Kreisstrom 5 an inertem Trägergas (z. B. Stickstoff oder Argon) im Gegenstrom zur Flüssigkeit durch den Verdampfer, wobei der Trägergasstrom mit Trioxan beladen wird. Der beladene Trägergasstrom wird durch entsprechende Stellung der Hähne 6 und 7 und korrespondierende Stellung der Hähne 8 und 9 auf einen der Wärmetauscher 10 und 11 geleitet, die hier als Desublimatoren ausgeführt sind. Unter der Voraussetzung einer genügend großen Temperaturdifferenz zwischen Verdampfer 2 und Wärmetauscher 10 bzw 11 fällt Trioxan in dem einen, mit Gas beaufschlagten Wärmetauscher in fester Form aus. Ist dieser eine Wärmetauscher beladen, so wird der Gasstrom über die Hähne 6 bis 9 auf den anderen Wärmetauscher geleitet. Im beladenen Wärmetauscher wird das Desublimat durch Temperaturerhöhung aufgeschmolzen und kann über die Hähne 12 bzw. 13 als Strom 14 bzw. 15 abgezogen werden. Ist der zweite Wärmetauscher beladen, so wird der Gasstrom wieder auf den zuvor entleerten Wärmetauscher geleitet, und im nun beladenen Wärmetauscher wird das Desublimat aufgeschmolzen und abgezogen.

Die Apparateverschaltung nach Figur 1 stellt nur eine denkbare Variante des erfindungsgemäßen Verfahrens dar. Wie oben beschrieben, sind beim erfindungsgemäßen Verfahren auch andere physikalische Prinzipien und Apparate zur Abtrennung des Trioxans durch Überführung von der flüssigen in die dampfförmige Phase und zurück zur flüssigen oder festen Phase einsetzbar.

Dadurch das bei dem erfindungsgemäßen Verfahren nur moderate Temperaturen erforderlich sind, läßt sich Wärme, die in anderen Prozeßschritten anfällt, hier in vorteilhafter Weise nutzen. Wird z. B., wie oben beschrieben, das erfindungsgemäße Verfahren mit einer vorausgehenden Trimerisierung von Formaldehyd aus der Gasphase kombiniert, so läßt sich die bei der Trimerisierung freiwerdende Wärme zur Beheizung des Verdunsters oder Verdampfers einsetzen. Befindet sich das aus dem Verdunster bzw. Verdampfer ablaufende Heizmedium auf einem genügend hohen Temperaturniveau, so läßt es sich weiter den Desublimatoren zum Zweck des Aufschmelzens von Produkt zuleiten. Auf diese Weise läßt sich die im Gesamtprozeß eingesetzte Wärmeenergie optimal nutzen.

Das erfindungsgemäße Verfahren wird nachfolgend anhand einiger experimenteller Untersuchungen illustriert. Der Versuchsaufbau erfolgte dabei gemäß der in Figur 1 dargestellten apparativen Variante.

Bei allen Versuchen kam als Verdampfer 2 ein Dünnschichtverdampfer DN25 mit einem Doppelmantel-Verdampferkörper aus Glas zum Einsatz; als Desublimatoren 10 und 11 wurden Doppelmantelrohre DN50 aus Glas verwendet.

Die Zuspeisung 1, bei allen Versuchen 400 ml/h, bestand aus einem flüssigen Gemisch enthaltend Trioxan, Cyclohexanol, Formaldehyd und Nebenkomponenten (vor allem Methanol und Wasser), wobei Formaldehyd darin überwiegend gebunden in Form von Cyclohexyl-Hemiformal vorlag (Ergebnisse siehe Tabelle 1). Vergleichsweise wurde auch eine Zuspeisung untersucht, die statt Cyclohexanol Wasser enthielt (Ergebnisse siehe Tabelle 2).

Als Trägergas wurde Stickstoff verwendet. Alle Versuche wurden bei Normaldruck bzw. leichtem Überdruck im Stickstoff-Kreislauf durchgeführt. Aus den ein- und austretenden Flüssigkeitsströmen wurden Proben entnommen und mit einem Gaschromatographen analysiert. Alle Prozentangaben hierzu sind als Massenprozente zu verstehen.

In den Tabellen 1 und 2 werden im weiteren die folgenden Abkürzungen benutzt:

| | | | |
|---|---|---|---|
| FA | Formaldehyd | TOX | Trioxan |
| MeOH | Methanol | CHOL | Cyclohexanol |
| H2O | Wasser | | |

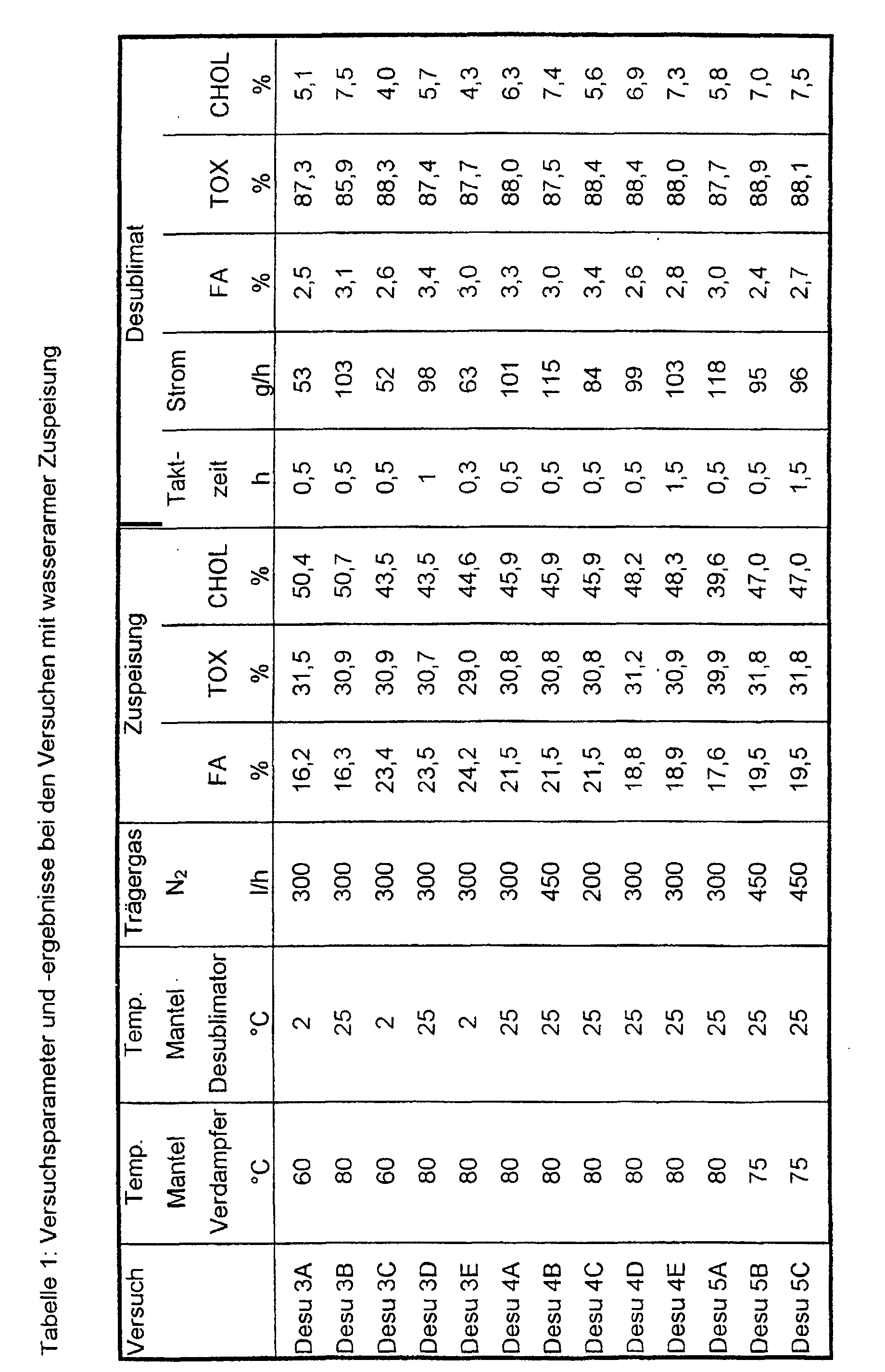

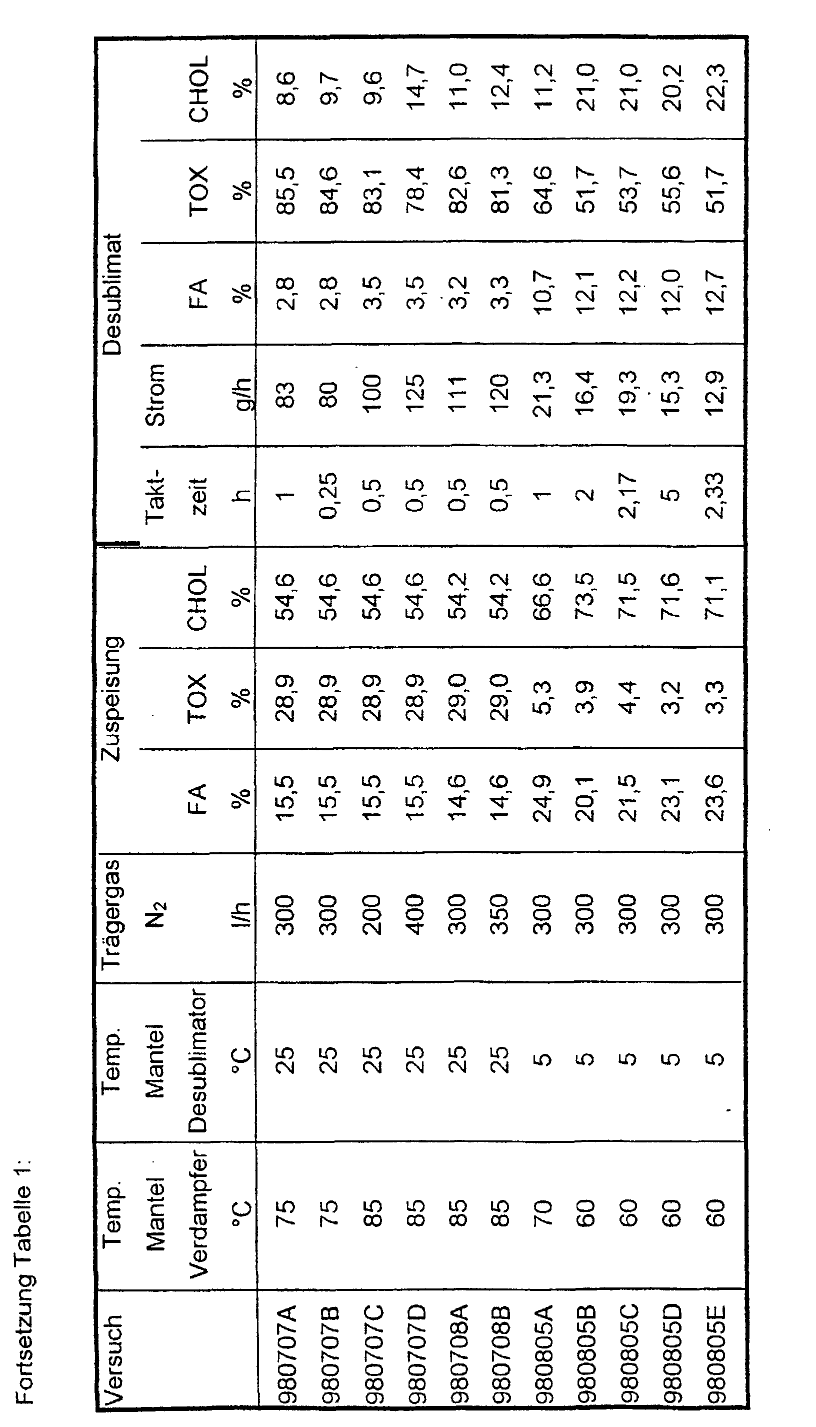

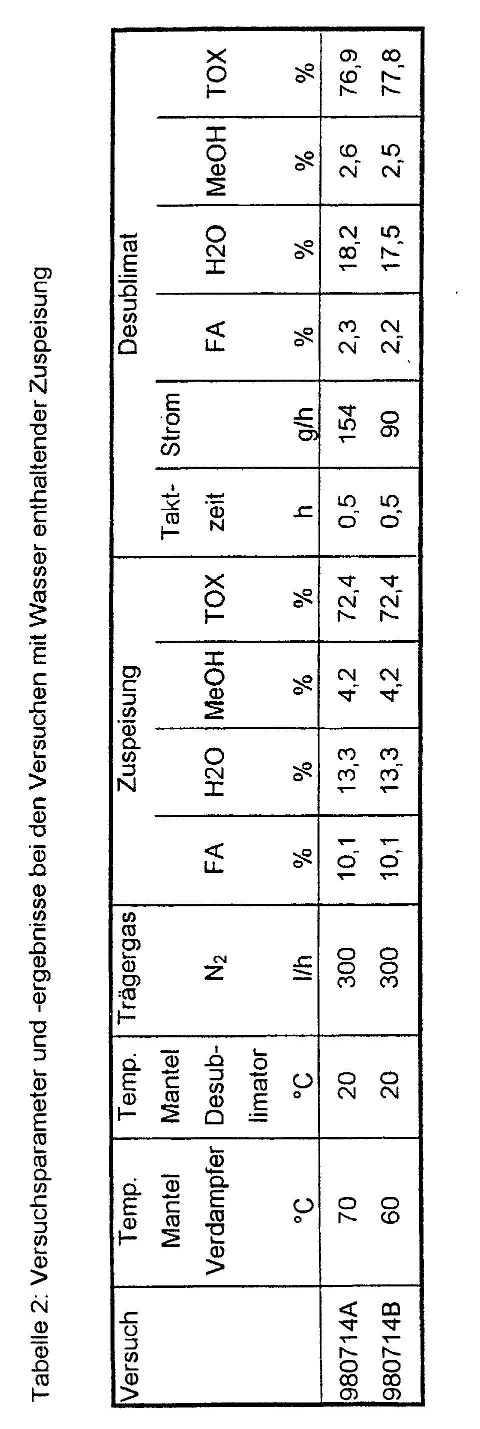

Bei allen Versuchen fiel das Trioxan als nadelförmiges Desublimat an; die Nadeln bildeten ein Geflecht sowie teilweise Brücken über den Querschnitt der Desublimatoren. Der im Kreis geführte Trägergasstrom wurde hierdurch in seiner Strömung nicht behindert. Durch das Geflecht wurde vielmehr in vorteilhafter Weise ein Austragen von festen Partikeln aus dem Desublimator mit dem Gasstrom verhindert, was den Einsatz eines nachgeschalteten Filters oder Abscheiders, wie es bei anderen Desublimatoren durchaus üblich ist, überflüssig machte.

Bei den oben beschriebenen Beispielen zu dem erfindungsgemäßen Verfahren erhielt man trotz des einfachen, einstufigen Aufbaus überraschend hohe Reinheiten und Ausbeuten. So ließ sich Trioxan aus wasserarmer Lösung von ca. 30 Gew.-% auf ca. 90 Gew.-% aufkonzentrieren, wobei Ausbeuten um die 80 % erreicht wurden. Im Desublimat ist der Formaldehyd gegenüber der Zuspeisung stark abgereichert, was für die Weiterverarbeitung des Desublimats vorteilhaft ist. Der Ablauf aus dem Verdampfer ist stark an Trioxan verarmt. So finden sich beispielsweise im Ablauf bei Versuch DESU4B nur 9,0 % TOX bei 28,4 % FA und 61,0 % CHOL; im Ablauf bei Versuch 980708A sind nur 6,7 % TOX bei 19,7 % FA und 72,2 % CHOL enthalten.

Des weiteren überraschte bei den Versuchen die Tatsache, daß beim Abschmelzen des Desublimats keine Vorlauffraktion, d.h. kein Kondensat aus einem Abschwitzvorgang abgezogen werden mußte, sondern daß vielmehr der gesamte Inhalt des Desublimators als Produkt verwendbar war. Dies führt zu einer vorteilhaften Erhöhung des Durchsatzes unter Verringerung der Taktzeiten. Wird hingegen eine Vorlauffraktion beim Abschmelzen abgezogen, so läßt sich die Reinheit des Produkts beim erfindungsgemäßen Verfahren in vorteilhafter Weise sogar noch steigern.

Anstelle der in den genannten Beispielen verwendeten Apparate sind für die Versuche auch alle anderen oben beschrieben Apparate denkbar. So ist beispielsweise statt des Dünnschichtverdampfers ein Fallfilmverdampfer einsetzbar. Die Desublimation läßt sich beispielsweise auch mit Rippenrohr-Wärmetauschern oder mit Fließbettapparaten durchführen.

### Bezugszeichenliste

- 1: Zuleitung des flüssigen Ausgangsgemisches
- 2: Verdampfer
- 3: Abzug der an Trioxan verarmten Flüssigkeit nach dem Verdampfer
- 4: Verdichter für Trägergas (Kreisgas)
- 5: Trägergasstrom (Kreisgas)
- 6: Hahn
- 7: Hahn
- 8: Hahn
- 9: Hahn
- 10: Wärmetauscher (Desublimator)
- 11: Wärmetauscher (Desublimator)
- 12: Hahn
- 13: Hahn
- 14: Abzug des Produktes aus dem Wärmetauscher (Desublimator)
- 15: Abzug des Produktes aus dem Wärmetauscher (Desublimator)

## Patentansprüche

1. Verfahren zur Gewinnung von Trioxan, wobei aus einem flüssigen Gemisch enthaltend Trioxan, Formaldehyd, Alkohol, aus dem Formaldehyd und dem Alkohol gebildete Hemiformale, übliche bei der Herstellung von Trioxan anfallende Nebenkomponenten sowie, zusätzlich oder alternativ zum Alkohol und den Hemiformalen, Wasser und aus Formaldehyd und Wasser gebildete Reaktionsprodukte das Trioxan durch Verdunstung oder Verdampfung in die Gasphase überführt und anschließend durch Kondensation in einen flüssigen Zustand gebracht und als Kondensat gewonnen oder durch Desublimation in einen festen Zustand gebracht und als Desublimat gewonnen wird.

2. Verfahren nach Anspruch 1, **dadurch gekennzeichnet, daß** das flüssige Gemisch im wesentlichen Trioxan, Formaldehyd, Alkohol, aus dem Formaldehyd und dem Alkohol gebildete Hemiformale, übliche bei der Herstellung von Trioxan anfallende Nebenkomponenten und 0 bis 5 Gew.-%, vorzugsweise 0 bis 3 Gew.-% Wasser enthält.

3. Verfahren nach Anspruch 1 oder 2, **dadurch gekennzeichnet, daß** das Kondensat oder das Desublimat einen Gehalt an Trioxan aufweist, der mindestens 2,5 mal so hoch ist wie der Trioxangehalt im flüssigen Gemisch.

4. Verfahren nach einem der Ansprüche 1 bis 3, **dadurch gekennzeichnet, daß** das Kondensat oder das Desublimat einen Gehalt an Trioxan von mindestens 80 Gew.-% aufweist.

5. Verfahren nach einem der Ansprüche 1 bis 4, **dadurch gekennzeichnet, daß** die Verdunstung oder Verdampfung bei einer Temperatur im Bereich von 20 bis 200°C erfolgt, wobei die Verdunstung oder Verdampfung wahlweise bei Unter-, Über- oder Normaldruck durchgeführt wird.

6. Verfahren nach Anspruch 5, **dadurch gekennzeichnet, daß** die Verdunstung oder Verdampfung bei einer Temperatur im Bereich von 50 bis 100°C erfolgt.

7. Verfahren nach einem der Ansprüche 1 bis 6, **dadurch gekennzeichnet. daß** eine Kondensation bei einer Temperatur im Bereich von 30 bis 113°C, bevorzugt bei einer Temperatur im Bereich von 30 bis 75°C erfolgt oder eine Desublimation bei einer Temperatur im Bereich von -20 bis 70°C, bevorzugt bei einer Temperatur im Bereich von 20 bis 40°C durchgeführt wird.

8. Verfahren nach einem der Ansprüche 1 bis 7, **dadurch gekennzeichnet. daß** es unter Verwendung eines Trägergases betrieben wird.

9. Verfahren nach einem der Ansprüche 1 bis 8, **dadurch gekennzeichnet. daß** die Verdunstung oder Verdampfung sowie die Kondensation oder Desublimation wahlweise jeweils in einem ein- oder mehrstufigen Prozeß erfolgt.

10. Verfahren nach einem der Ansprüche 1 bis 9, **dadurch gekennzeichnet, daß** die Verdunstung oder Verdampfung in mindestens einer Apparatur ausgewählt unter Verdampfern, Kolonnen, Rührgefäßen, Blasensäulen und Flash-Apparaturen und die Kondensation oder Desublimation in mindestens einer Apparatur ausgewählt unter Wärmetauschern, Fließbettapparaten, Wirbelschichtapparaten, Blasensäulen und Rührgefäßen durchgeführt wird.

## Claims

1. A process for the recovery of trioxane, in which the trioxane is transferred from a liquid mixture comprising trioxane, formaldehyde, alcohol, hemiformals formed from the formaldehyde and the alcohol, usual secondary components arising in the preparation of trioxane, and, in addition or as an alternative to the alcohol and the hemiformals, water and reaction products formed from formaldehyde and water, into the gas phase by volatilization or evaporation and subsequently converted into a liquid state by condensation and isolated as condensate or converted into a solid state by desublimation and isolated as desublimate.

2. The process as claimed in claim 1, wherein the liquid mixture essentially comprises trioxane, formaldehyde, alcohol, hemiformals formed from the formaldehyde and the alcohol, usual secondary components arising in the preparation of trioxane, and from 0 to 5% by weight, preferably from 0 to 3% by weight, of water.

3. The process as claimed in claim 1 or 2, wherein the condensate or the desublimate has a trioxane content which is at least 2.5 times as high as the trioxane content in the liquid mixture.

4. The process as claimed in one of claims 1 to 3, wherein the condensate or the desublimate has a trioxane content of at least 80% by weight.

5. The process as claimed in one of claims 1 to 4, wherein the vaporization or evaporation is carried out at a temperature in the range from 20 to 200°C, the vaporization or evaporation optionally being carried out at reduced pressure, superatmospheric pressure or atmospheric pressure.

6. The process as claimed in claim 5, wherein the vaporization or evaporation is carried out at a temperature in the range from 50 to 100°C.

7. The process as claimed in one of claims 1 to 6, wherein a condensation is carried out at a temperature in the range from 30 to 113°C, preferably at a temperature in the range from 30 to 75°C, or a desublimation is carried out at a temperature in the range from -20 to 70°C, preferably at a temperature in the range from 20 to 40°C.

8. The process as claimed in one of claims 1 to 7, which is carried out using a carrier gas.

9. The process as claimed in one of claims 1 to 8, wherein the vaporization or evaporation and the condensation or desublimation is optionally each carried out in a one-step or multistep process.

10. The process as claimed in one of claims 1 to 9, wherein the vaporization or evaporation is carried out in at least one apparatus selected from evaporators, columns, stirred vessels, bubble-tray columns and flash apparatuses, and the condensation or desublimation is carried out in at least one apparatus selected from heat exchangers, fluid-bed apparatuses, fluidized-bed apparatuses, bubble-tray columns and stirred vessels.

## Revendications

1. Procédé pour l'obtention de trioxanne, dans lequel, à partir d'un mélange liquide contenant du trioxanne, du formaldéhyde, un alcool, des hémiformals formés à partir du formaldéhyde et de l'alcool, des composants secondaires usuels apparaissant dans la préparation du trioxanne ainsi que, en plus ou au lieu de l'alcool et des hémiformals, de l'eau et des produits de réaction formés à partir de formaldéhyde et d'eau, le trioxanne est converti en la phase gazeuse par évaporation ou vaporisation et ensuite mis en un état liquide par condensation et obtenu sous forme de condensat ou mis en un état solide par désublimation et obtenu sous forme de désublimat.

2. Procédé selon la revendication 1, **caractérisé en ce que** le mélange liquide contient essentiellement du trioxanne, du formaldéhyde, un alcool, des hémiformals formés à partir du formaldéhyde et de l'alcool, des composants secondaires usuels apparaissant dans la préparation du trioxanne et de 0 à 5 % en poids, de préférence, de 0 à 3 % en poids d'eau.

3. Procédé selon la revendication 1 ou 2, **caractérisé en ce que** le condensat ou le désublimat présente une teneur en trioxanne qui est au moins 2,5 fois supérieure à la teneur en trioxanne du mélange liquide.

4. Procédé selon l'une quelconque des revendications 1 à 3, **caractérisé en ce que** le condensat ou le désublimat présente une teneur en trioxanne d'au moins 80 % en poids.

5. Procédé selon l'une quelconque des revendications 1 à 4, **caractérisé en ce que** l'évaporation ou la vaporisation s'effectue à une température dans la plage de 20 à 200°C, l'évaporation ou la vaporisation étant effectuée, au choix, sous pression réduite, sous pression ou sous la pression normale.

6. Procédé selon la revendication 5, **caractérisé en ce que** l'évaporation ou la vaporisation s'effectue à une température dans la plage de 50 à 100°C.

7. Procédé selon l'une quelconque des revendications 1 à 6, **caractérisé en ce qu'**une condensation est effectuée à une température dans la plage de 30 à 113°C, de préférence à une température dans la plage de 30 à 75°C ou une désublimation est effectuée à une température dans la plage de -20 à 70°C, de préférence à une température dans la plage de 20 à 40°C.

8. Procédé selon l'une quelconque des revendications 1 à 7, **caractérisé en ce qu'**on l'effectue avec utilisation d'un gaz vecteur.

9. Procédé selon l'une quelconque des revendications 1 à 8, **caractérisé en ce que** l'évaporation ou la condensation ainsi que la condensation ou la désublimation s'effectuent chacune, au choix, dans un processus en une ou plusieurs étapes.

10. Procédé selon l'une quelconque des revendications 1 à 9, **caractérisé en ce que** l'évaporatio ou la vaporisation est effectuée dans au moins un appareil choisi parmi des vaporiseurs, des colonnes, des récipients à agitation, des colonnes à barbotage et des appareils instantanés et la condensation ou la désublimation est effectuée dans au moins un appareil choisi parmi des échangeurs thermiques, des appareils à lit fluidisé, des appareils à couche tourbillonnaire, des colonnes à barbotage et des récipients à agitation.
